# EUROPÄISCHE PATENTSCHRIFT

(11) **EP 2 120 820 B1**
(45) Veröffentlichungstag und Bekanntmachung des Hinweises auf die Patenterteilung: **04.04.2012**
(21) Anmeldenummer: 07701878.6
(22) Anmeldetag: 14.02.2007
(51) Int. Cl.: A61F 9/008, A61F 9/009, A61F 9/01

(54) **OPHTHALMOLOGISCHE VORRICHTUNG FÜR DIE AUFLÖSUNG VON AUGENGEWEBE**
OPHTHALMOLOGICAL DEVICE FOR RESOLVING EYE TISSUE
DISPOSITIF OPHTHALMOLOGIQUE POUR LA DISSOLUTION DU TISSU OCULAIRE

(43) Veröffentlichungstag der Anmeldung: 25.11.2009
(73) Patentinhaber: Ziemer Holding AG, 2562 Port (CH)
(72) Erfinder: RATHJEN, Christian, 28197 Bremen (DE)
(74) Vertreter: Rentsch Partner AG
(86) Internationale Anmeldenummer: PCT/CH2007/000078
(87) Internationale Veröffentlichungsnummer: WO 2008/098381

(56) Entgegenhaltungen:
- EP-A- 1 731 120
- EP-A1- 1 486 185
- WO-A-89/06519

## Beschreibung

### Technisches Gebiet

Die vorliegende Erfindung betrifft eine ophthalmologische Vorrichtung für die Auflösung von Augengewebe. Die Erfindung betrifft insbesondere eine ophthalmologische Vorrichtung, welche eine Basisstation mit einer Lichtquelle zur Erzeugung von Lichtpulsen, einen mittels eines Tragarms an der Basisstation angebrachten, auf ein Auge aufsetzbaren Applikationskopf mit einem Lichtprojektor zur fokussierten Projektion der Lichtpulse, sowie ein optisches Übertragungssystem zur Übertragung der Lichtpulse von der Basisstation durch den Tragarm zum Applikationskopf umfasst.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem computergesteuerten Excimerlaser abgetragen wird, nachdem zuvor ein Hornhautlappen teilweise abgetrennt und weggeklappt wird. Zur Erzeugung des Hautlappens werden mechanische Mikrokeratome eingesetzt, bei denen ein angetriebenes Messer den Hautlappen schneidet. Neuerdings können solche Hautlappen auch mit stark fokussierten Femtosekundenlaserpulsen geschnitten werden, die Pulsbreiten von typisch 100fs bis 1000fs (1fs=10⁻¹⁵s) aufweisen. Neben der LASIK gibt es weitere Prozeduren zur refraktiven Korrektur, die an der Hornhaut mit Hilfe von Femtosekundenlasern durchgeführt werden.

Ein solches System wird z. B. von der Firma IntraLase Corp, in Irvine, California, USA, unter dem Namen Pulsion FS Laser angeboten. In diesem System befindet sich die Lichtquelle (Femtolaser) in einer Basisstation und ist über einen Spiegelgelenkarm mit einem optischen Applikationskopf verbunden. Neben dem optischen Übertragungssystem, einem Ablenkungssystem (Scanner) und dem Lichtprojektor, umfasst der Applikationskopf zudem Beobachtungseinrichtungen, wie Kamera und Operationsmikroskop, und weist damit ein beträchtliches Gewicht von mehreren Kilogramm auf. Zum Ausrichten und Applizieren des Lichtprojektors auf ein Auge des Patienten wird der gesamte Applikationskopf über translatorische Antriebe bewegt. Der Patient befindet sich dabei auf einer Liege und bewegt sich nicht. Aus Gewichtsgründen kann das Applizieren nicht von Hand ausgeführt werden und es sind motorische Antriebe erforderlich. Um zu grosse Kräfte auf den Patienten zu verhindern, verfügt das Lasersystem über Kraftmesssysteme in der vertikalen Applikationsrichtung.

Auch die Firmen Zeiss Meditec AG, mit Visumax, und 20/10 Perfect Vision optische Geräte GmbH, mit Femtec, bieten Systeme zum Schneiden des Hautlappens mit fokussierten Femtosekundenlaserpulsen an. Bei diesen Systemen ist der Lichtprojektor fest mit der Basisstation verbunden. Bei diesen Systemen wird der Patient in der horizontalen Ebene sowie vertikal mit Hilfe eines Patientenbetts bezüglich des Lichtprojektors ausgerichtet. Somit können auch diese Systeme nicht manuell an das Auge des Patienten aufgesetzt werden. Zudem muss das spezielle Patientenbett in das Sicherheitssystem des Lasersystems integriert werden, um unkontrollierte Bewegungen des Betts zu verhindern. Das Bett ist damit Teil des Lasersystems und erhöht somit die Systemkosten und den Platzbedarf. Eine eigene Wahl des Betts durch den Benutzer des Systems ist nicht möglich.

Bei den vorgenannten Systemen wird das Andocken des Lichtprojektors an das Patientenauge zunächst durch Ausrichten in der horizontalen Ebene in der x- und y-Richtung (Zentrieren des Auges) und dann durch Absenken in vertikaler z-Richtung durchgeführt.

In EP 1731120 wird ein System beschrieben, bei welchem der Applikationskopf über einen aus mehreren Armelementen und Gelenken bestehenden Spiegelgelenkarm flexibel an der Basisstation angebracht ist und eine manuelle Applikation des Applikationskopfs und Lichtprojektors auf das Auge eines Patienten ermöglicht. Um das Gewicht des Applikationskopfs für die manuelle Applikation mittels des Spiegelgelenkarms zu ermöglichen weist der Lichtprojektor gegenüber den vorgenannten Systemen kleiner und damit leichter dimensionierte Linsensysteme auf. Um trotz des kleiner dimensionierten Linsensystems zum Schneiden des Gewebelappens auf dem Auge ein ausgedehntes Bearbeitungsgebiet mit fokussierten Laserpulsen bearbeiten zu können, verfügt der Applikationskopf zudem über Bewegungstreiber zur Bewegung des Lichtprojektors in einer Vorschubrichtung und in einer ersten Abtastrichtung. Eine optische Ablenkung in einer vertikalen Abtastrichtung ist dabei allerdings nicht möglich. Das begrenzte Arbeitsgebiet erlaubt nur eine schnelle Positionierung der Laserpulse innerhalb des Bearbeitungsgebiets. Damit sind einer flexiblen Positionierung der Laserpulse über dem gesamten Auge Grenzen gesetzt.

### Darstellung der Erfindung

Es ist eine Aufgabe der vorliegenden Erfindung, eine neue ophthalmologische Vorrichtung für die Auflösung von Augengewebe vorzuschlagen, welche mindestens gewisse Nachteile des Stands der Technik nicht aufweist. Es ist insbesondere eine Aufgabe der vorliegenden Erfindung eine ophthalmologische Vorrichtung für die Auflösung von Augengewebe vorzuschlagen, welche eine manuelle Applikation des Lichtprojektors auf ein Auge eines Patienten ermöglicht dabei aber einen Lichtprojektor mit einer Optik hoher numerischer Apertur zulässt, welche die fokussierte Projektion von Laserpulsen im gesamten einsehbaren Augenbereich sowohl in horizontaler als auch in vertikaler Richtung zulässt.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die ophthalmologische Vorrichtung umfasst eine Basisstation mit einer Lichtquelle zur Erzeugung von Lichtpulsen, einen an der Basisstation angebrachten Tragarm, einen am Tragarm angebrachten und auf ein Auge aufsetzbaren Applikationskopf mit einem Lichtprojektor zur fokussierten Projektion der Lichtpulse für eine punktuelle Auflösung von Augengewebe, sowie ein optisches Übertragungssystem zur Übertragung der Lichtpulse von der Basisstation durch den Tragarm zum Applikationskopf.

Die oben genannten Ziele werden durch die vorliegende Erfindung insbesondere dadurch erreicht, dass der Tragarm starr ausgeführt ist und an einem Ende ein Drehgelenk mit einer horizontal ausgerichteten Drehachse aufweist, wobei das Drehgelenk so angebracht ist, dass der Applikationskopf mit einer um die Drehachse verlaufenden Drehung (Drehbewegung) auf das Auge aufsetzbar ist. Der starre, d.h. einteilig und in sich unbeweglich, ausgeführte Tragarm ermöglicht eine stabile und einfache Verbindung des Applikationskopfs mit der Basisstation. Insbesondere ermöglicht der starre Tragarm das optische Übertragungssystem einfacher auszugestalten als beispielsweise bei einem mehrteiligen Spiegelgelenkarm. Das horizontal an einem Ende des starren Tragarms angeordnete Drehgelenk ermöglicht die Applikation des Applikationskopfs respektive des Lichtprojektors auf das Auge in vertikaler Richtung über eine vorzugsweise mechanisch ausgeführte Rotationsbewegung durchzuführen, wobei der Applikationskopf auf einem kinematisch erzeugten Bogen geführt wird, der eine vertikale Bewegungskomponente zum Aufsetzen auf dem Auge erlaubt. Der Vorteil der Rotationsbewegung ist ihre einfache Ausführung und geringe mechanische Reibung. Der Tragarm und das Drehgelenk ermöglichen somit ein kontrolliertes manuelles Andocken, d.h. Applizieren des Applikationskopfs respektive des Lichtprojektors auf das Auge, wobei der Benutzer seine taktilen Sinne vorteilhaft nutzen kann um über die taktile Kraft- und Verschiebungsrückkopplung seinen Kraft- respektive Verschiebungsaufwand zu steuern. Weil aktive Systeme für die Kraftaufwendung und Kraftbegrenzung entbehrlich sind; wird eine erhöhte Sicherheit nur durch die Verwendung passiver Systemelemente erreicht. Auf Grund des einfach gestalteten Tragarms und der nicht oder mit geringerer Anzahl erforderlichen Aktoren und Sensoren werden eine weiter vereinfachte Bauform und damit geringere Kosten ermöglicht. Überdies kann die Baugrösse der Komponenten, die sich direkt über dem Patienten befinden, reduziert werden.

Vorzugsweise ist das Drehgelenk am Ende des Tragarms angeordnet, das von der Basisstation abgewandt ist, und der Applikationskopf ist über das Drehgelenk beweglich mit dem Tragarm verbunden. Durch die bewegliche Anordnung des Applikationskopfs am starren Tragarm mittels eines Drehgelenks wird die Grösse und das Gewicht des manuell zu applizierenden Systemteils reduziert, was die manuelle Applikation weiter erleichtert und kontrollierbarer macht.

In einer alternativen Ausführung ist der Applikationskopf fest mit dem Tragarm verbunden und das Drehgelenk ist am Ende des Tragarms angeordnet, das der Basisstation zugewandt ist, so dass der Tragarm und damit der daran befestigte Applikationskopf über das Drehgelenk beweglich mit der Basisstation verbunden ist. Der Vorteil eines verlängerten Dreharms besteht darin, dass die bei der Applikation ausgeführte Drehung einen grösseren Radius aufweist und damit für die vertikale Auslenkung einen kleineren Drehwinkel benötigt, was wiederum geringere Abweichungen von einer vertikalen Applikationsachse bedeutet.

In einer weiteren Ausführungsvariante umfasst die Vorrichtung Positionierungsmittel zum rotatorischen und/oder translatorischen Bewegen des Tragarms parallel zu einer Positionierungsebene für die Positionierung des Applikationskopfs über das Auge. Die Positionierungsmittel umfassen beispielsweise Führungsschienen und/oder Antriebsmittel für translatorische Bewegungen in einer horizontalen Positionierungsebene und/oder Drehgelenke, die eine Drehung (Drehbewegung) um eine vertikal verlaufende Drehachse ermöglichen.

In einer Ausführungsvariante umfasst die Vorrichtung im Tragarm angeordnete Strahlablenkungsmittel zur Ablenkung der Lichtpulse in mindestens zwei Abtastrichtungen, und der Lichtprojektor ist so dimensioniert, dass abgelenkte Lichtpulse ohne mechanische Bewegung des Lichtprojektors über einen gesamten Arbeitsbereich von vorzugsweise 11 mm Durchmesser zur Auflösung von Augengewebe fokussiert projizierbar sind.

In einer weiteren Ausführungsvariante weist der Applikationskopf ein Sichtfenster auf, das eine Aufsicht auf das Auge in Projektionsrichtung des Lichtprojektors ermöglicht. Das Sichtfenster ermöglicht optische Sehhilfe- oder Messmodule, z.B. Kameras, Mikroskope oder Messgeräte, die beweglich mit der Basisstation verbunden sind und die in Projektionsrichtung des Lichtprojektors ausgerichtet über das Sichtfenster schwenkbar sind, eine Aufsicht auf das Auge zu verschaffen, während der Applikationskopf auf das Auge aufgesetzt ist bzw. wird. Andererseits können die optischen Sehhilfe- oder Messmodule weggeschwenkt werden, wenn Sie nicht benötigt werden. Durch die Möglichkeit, optische Sehhilfe- oder Messmodule je nach Bedarf in die Sehachse über dem Auge hinein- respektive herauszuschwenken, können die Baugrösse und damit das Gewicht des Applikationskopfs weiter reduziert und die Handbarkeit verbessert werden.

In einer Ausführungsvariante sind Mittel zur Gewichtskompensation mit dem Applikationskopf verbunden, um den Applikationskopf über der Drehachse teilweise so auszubalancieren, dass der Applikationskopf mit einer vom Eigengewicht des Applikationskopfs reduzierten Applikationskraft auf das Auge aufsetzbar ist. Durch die nur teilweise austarierte Konfiguration der in der Drehbewegung involvierten Massen (Applikationskopf und je nach Variante der Tragarm) kann die Applikationskraft auf das Auge durch die Schwerkraft erzeugt werden.

In einer weiteren Ausführungsvariante umfasst der Lichtprojektor ein erstes Linsensystem in einem in Projektionsrichtung ausgerichteten Projektionsteil des Applikationskopfs, und der Lichtprojektor umfasst ein zweites Linsensystem in einem vom Projektionsteil abgewinkelten, dem Tragarm zugewandten Zuführteil des Applikationskopfs, wobei das erste und das zweite Linsensystem über einen Umlenkspiegel gekoppelt sind. Die Anordnung der Linsensysteme aufgeteilt in den Projektions- und Zuführteil ermöglicht eine Reduktion der Bauhöhe des Lichtprojektors und damit des Applikationskopfs. Weiterhin lassen sich externe Hilfsmittel wie Mikroskope oder Messgeräte einfacher anpassen.

In einer Ausführungsvariante umfasst der Applikationskopf einen in Projektionsrichtung ausgerichteten Projektionsteil und einen vom Projektionsteil abgewinkelten, dem Tragarm zugewandten Zuführteil. Der Projektionsteil ist drehbar mit dem Zuführteil verbunden, z.B. axial um eine durch den Zuführteil verlaufende Längsachse und/oder um eine Querachse, die senkrecht zur Längsachse des Zuführteils verläuft. Die drehbare Verbindung des Projektionsteils mit dem Zuführteil ermöglicht eine Feinausrichtung des Lichtprojektors und daran angebrachter Befestigungsmittel zum Auge.

In einer weiteren Ausführungsvariante umfasst die Vorrichtung eine Verriegelung zum Fixieren respektive Lösen einer Drehstellung des Applikationskopf um die Drehachse.

In einer Ausführungsvariante umfasst die Vorrichtung Höhenbestimmungsmittel zum Ermitteln einer vertikalen Lage des Auges und die Basisstation umfasst Höhenpositionierungsmittel zum Einstellen einer vertikalen Grundposition des Tragarms. Durch die Einstellung einer vertikalen Grundposition des Tragarms kann die zum vertikalen Aufsetzen des Applikationskopfs auf das Auge benötigte Drehbewegung reduziert und damit in einem näher an der vertikalen Bewegungskomponente liegenden Bereich gehalten werden, was einerseits den notwendigen Bewegungshub zum Andocken des Applikationskopfs reduziert und andererseits die horizontale Ausrichtung von Applikationskopf und Auge (Zentrierung) erleichtert.

In weiteren Ausführungsvarianten umfasst der Applikationskopf einen oder mehrere Griffe oder Griffstrukturen zur manuellen Handhabung, einen auf das Auge aufsetzbaren, mindestens stellenweise lichtdurchlässigen Kontaktkörper, welcher so ausgebildet und angeordnet ist, dass er einen im aufgesetzten Zustand kontaktierten Bereich des Auges vorzugsweise äquidistant zu einer Arbeitsfläche setzt, sowie Befestigungsmittel zum Fixieren des Applikationskopfs z.B. durch Unterdruck am Auge. Die Lichtquelle umfasst vorzugsweise einen Femtosekundenlaser.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt schematisch eine seitliche Ansicht der ophthalmologischen Vorrichtung, die eine einteilige Ausführung einer Basisstation mit einem Tragarm umfasst, an welchem ein Applikationskopf drehbar angebracht ist.
Figur 2 zeigt schematisch eine seitliche Ansicht einer ophthalmologischen Vorrichtung, die einen in einer horizontalen Positionierungsebene translatorisch zur Basisstation bewegbaren Tragarm umfasst, an welchem der Applikationskopf drehbar angebracht ist.
Figur 3 zeigt schematisch eine Aufsicht einer ophthalmologischen Vorrichtung, die einen in der Positionierungsebene translatorisch zur Basisstation bewegbaren Tragarm umfasst, an welchem der Applikationskopf drehbar angebracht ist.
Figur 4 zeigt schematisch eine Aufsicht einer ophthalmologischen Vorrichtung, die einen in der Positionierungsebene translatorisch und rotatorisch zur Basisstation bewegbaren Tragarm umfasst, an welchem der Applikationskopf drehbar angebracht ist.
Figur 5 zeigt schematisch eine Aufsicht einer ophthalmologischen Vorrichtung, die einen in der Positionierungsebene um zwei vertikale Drehachsen rotatorisch zur Basisstation bewegbaren Tragarm umfasst, an welchem der Applikationskopf drehbar angebracht ist.
Figur 6 zeigt schematisch eine Aufsicht einer weiteren ophthalmologischen Vorrichtung, die einen in der Positionierungsebene um zwei vertikale Drehachsen rotatorisch zur Basisstation bewegbaren Tragarm umfasst, an welchem der Applikationskopf drehbar angebracht ist.
Figur 7 zeigt schematisch eine seitliche Ansicht des Applikationskopfs, der ein Linsensystem in einem Zuführteil zum Tragarm und ein weiteres Linsensystem in einem abgewinkelten Projektionsteil aufweist.
Figur 8 zeigt schematisch eine seitliche Ansicht des Applikationskopfs, der ein Sichtfenster über dem Projektionsteil aufweist, an das ein optisches Sehhilfe oder Messmodul entfernbar angekoppelt ist.
Figur 9 zeigt schematisch eine seitliche Ansicht des Applikationskopfs, der einen drehbar mit dem Zuführteil verbundenen Projektionsteil umfasst.

### Wege zur Ausführung der Erfindung

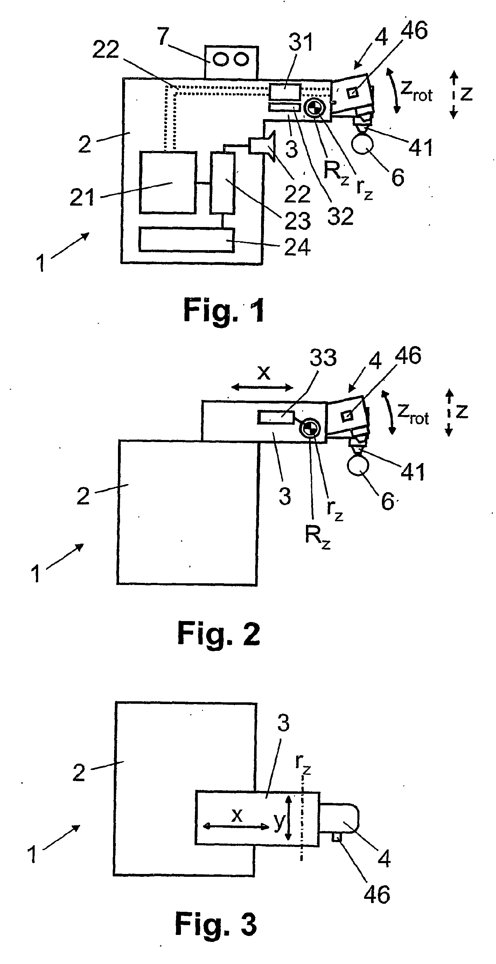

In den Figuren 1 bis 6 bezeichnet das Bezugszeichen 1 eine ophthalmologische Vorrichtung für die Auflösung von Augengewebe. Die ophthalmologische Vorrichtung 1 umfasst jeweils eine Basisstation 2 und einen daran angebrachten, in sich starren Tragarm 3. Am Tragarm 3 ist jeweils ein Applikationskopf 4 fest oder über ein horizontal ausgerichtetes Drehlager R_{z} angebracht. In der Figur 1 ist zudem ein optionales, fest mit dem Tragarm 3 verbundenes optisches Sehhilfe-, Bilderfassungs- und/oder Messmodul 7, dargestellt, z.B. ein Monitor und/oder ein Mikroskop zur Betrachtung des Applikationsvorgangs (Andocken) und der Behandlung. In den Ausführungsvarianten gemäss den Figuren 1 bis 6 ist der Applikationskopf 4 über das Drehgelenk R_{z} um die horizontale Drehachse r_{z} drehbar mit dem Tragarm 3 verbunden. Das Drehgelenk R_{z} ist jeweils am Ende des Tragarms 3 angeordnet, das von der Verbindung des Tragarms 3 mit der Basisstation 2 abgewandt ist. Wie in den seitlichen Ansichten der Figuren 1 und 2 schematisch dargestellt ist, ist das Drehgelenk R_{z} so angeordnet und ausgeführt, dass durch die Drehung zᵣₒₜ des Applikationskopfs 4 um die Drehachse r_{z} eine vertikale Bewegungskomponente in z-Richtung zum Absenken und Aufsetzen des Applikationskopfs 4 auf ein Auge 6 ausgeführt werden kann. Zur manuellen Handhabung sind am Applikationskopf 4 Griffe und/oder Griffstrukturen 46 angebracht. Um die Massenträgheit klein zu halten und die Anzahl der bewegten Komponenten zu reduzieren, ist die Drehachse r_{z} dicht am Patienten angeordnet. Der Fachmann wird verstehen, dass die Drehung des Applikationskopfs 4 um die Drehachse r_{z} auch z.B. mittels einer Parallelogrammführung erfolgen kann, wobei dazu weitere Drehgelenke R_{z} eingesetzt werden.

Obwohl dies nur in der Figur 1 schematisch dargestellt ist, umfasst die ophthalmologische Vorrichtung 1 jeweils eine in der Basisstation 2 angeordnete Lichtquelle 21 (Laserquelle) zur Erzeugung von Lichtpulsen, insbesondere einen Femtosekundenlaser zur Erzeugung von Femtosekundenlaserpulsen, sowie ein optisches Übertragungssystem 22 zur Übertragung der Lichtpulse von der Basisstation 2 durch den Tragarm 3 zum Applikationskopf 4. Vorzugsweise umfasst die ophthalmologische Vorrichtung 1 zudem eine Verriegelung 32 zum Fixieren respektive Lösen einer Drehstellung des Applikationskopfs 4 um die Drehachse r_{z}, beispielsweise eine Reibkupplung.

In das optische Übertragungssystem 22 sind Strahlablenkungsmittel 31 eingefügt, die zur Ablenkung der Lichtpulse in mindestens zwei Abtastrichtungen eingerichtet sind. Ein für die Strahlablenkungsmittel 31 geeigneter Scanner wird beispielsweise in EP 1731120 beschrieben. Weiterhin eignen sich Galvanoscanner oder akustooptische Modulatoren. Die Strahlablenkungsmittel 31 sind vorzugsweise im Tragarm 3 angeordnet um die Distanz zum Applikationskopf 4 gering zu halten. Der Applikationskopf 4 umfasst einen Lichtprojektor 41 mit einem Linsensystem 42, 43 zur fokussierten Projektion der Lichtpulse in und/oder auf das Auge 6 um das Augengewebe punktuell aufzulösen. Die abgelenkten Lichtpulse werden durch das optische Übertragungssystem 22 weiter von den Strahlablenkungsmitteln 31 zum Lichtprojektor 41 übertragen. Die Strahlablenkungsmittel 31 und der Lichtprojektor 41 sind eingerichtet ein über den gesamten einsehbaren Augenbereich ausgedehntes Arbeitsbereich zusammenhängend abzutasten und fokussiert zu bearbeiten, so dass zusammenhangende Schnitte im Augengewebe, insbesondere in der Comea (Augenhomhaut) durchgeführt werden können. Die Strahlablenkungsmittel 31 und der Lichtprojektor 41 ermöglichen nicht nur das fokussierte Abtasten eines planen, beispielsweise horizontalen Arbeitsbereichs, sondern durch gezieltes vertikales Positionieren des Fokus auch dreidimensional definierte Arbeitsbereiche, z.B. vertikale und gebogene Schnittflächen.

Obwohl dies in den Figuren nicht dargestellt ist, soll angeführt werden, dass der Applikationskopf 4 einen auf das Auge 6 aufsetzbaren, mindestens stellenweise lichtdurchlässigen Kontaktkörper umfasst, welcher so ausgebildet und angeordnet ist, dass er einen im aufgesetzten Zustand kontaktierten Bereich des Auges 6 vorzugsweise äquidistant zu einer Arbeitsfläche setzt, sowie Befestigungsmittel zum Fixieren des Applikationskopfs 4 durch Unterdruck am Auge 6. Kontaktkörper können z.B. plan oder sphärisch ausgeführt werden.

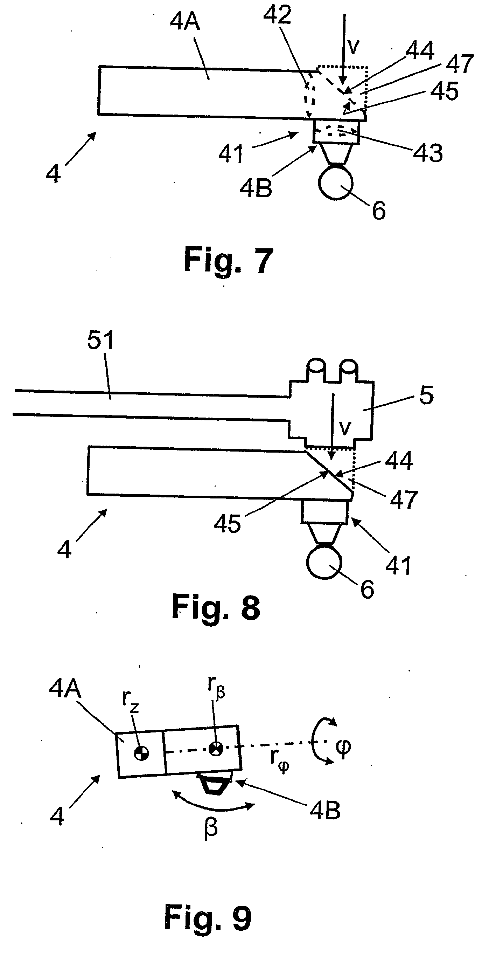

Wie in der Figur 7 schematisch dargestellt ist, weist der Applikationskopf 4 in einer mit den Figuren 1-6 kombinierbaren Ausführungsvariante einen mit dem Tragarm 3 verbundenen Zuführteil 4A und einen vom Zuführteil 4A abgewinkelten Projektionsteil 4B. In dieser Ausführungsvariante umfasst der Lichtprojektor 41 ein erstes Linsensystem 43 im Projektionsteil 4B und ein zweites Linsensystem 42 im Zuführteil 4A. Das erste Linsensystem 43 und das zweite Linsensystem 42 sind über den Umlenkspiegel 45 gekoppelt. Vorzugsweise weist der Applikationskopf 4 zudem ein Sichtfenster 44 auf, das eine Aufsicht auf das Auge 6 in der Projektionsrichtung v ermöglicht. Das Sichtfenster 44 ist beispielsweise so ausgeführt, dass der Umlenkspiegel 45 lichtdurchlässig für die Beobachtungswellenlänge ausgestaltet ist. Das Bezugszeichen 47 bezieht sich auf Kopplungsmittel, welche nachfolgend detaillierter beschrieben werden.

Wie in der Figur 8 schematisch dargestellt ist, ermöglicht das Sichtfenster 44 die optische Ankopplung von optischen Sehhilfe-, Bilderfassungs- und Messmodulen 5, z.B. eine Aufsichtskamera mit optionalem schwenkbaren Monitor, die beispielsweise mittels eines Modulträgers 51 an der Basisstation 2 um eine vertikal ausgerichtete Drehachse drehbar angebracht sind. Diese Module können somit über den Applikationskopf 4 ein- und weggeschwenkt und ihre optischen Achsen auf die optische Projektionsachse v des Lichtprojektors 41 ausgerichtet werden. Die Module können zudem über die Kopplungsmittel 47, beispielsweise ein lösbarer Schnappverschluss oder ein Bajonettverschluss, entfernbar mit dem Applikationskopf 4 mechanisch verbunden werden.

In einer optionalen, mit den Figuren 1-8 kombinierbaren und in der Figur 9 schematisch dargestellten Ausführungsvariante, ist der Projektionsteil 43 drehbar am Zuführteil 42 angebracht. In der Variante nach Figur 9 weist der Applikationskopf 4 ein Drehgelenk R_{ϕ} auf und der Projektionsteil 43 ist mit dem Drehwinkel ϕ um eine durch den Zuführteil 42 verlaufende Längsachse r_{ϕ} drehbar verbunden. Zudem weist der Applikationskopf 4 ein Drehgelenk R_{β} auf und der Projektionsteil 43 ist mit dem Drehwinkel β um eine zur Längsachse r_{ϕ} senkrecht verlaufenden Querachse r_{β} drehbar mit dem Zuführteil 42 verbunden.

In der Ausführungsvariante nach Figur 1 ist der Tragarm 3 fest mit der Basisstation 2 verbunden, zum Beispiel sind die Basisstation 2 und der Tragarm 3 einteilig oder einstückig als Gesamteinheit mit einem gemeinsamen Gehäuse ausgeführt. In dieser Variante erfolgt die gegenseitige Ausrichtung von Auge 6 und Lichtprojektor 41, d.h. die Zentrierung des Lichtprojektors 41 zum Auge 6, durch Verschieben des Patientenbetts oder der Basisstation 2 in x- und y-Richtung einer horizontalen Positionierungsebene (in der Regel parallel zur Grundfläche auf der die Basisstation 2 angeordnet ist).

In der Ausführungsvariante gemäss den Figuren 2 und 3 ist der Tragarm 3 so beweglich mit der Basisstation 2 verbunden, dass der Tragarm 3 für die Ausrichtung des Lichtprojektors 41 zum Auge 6 translatorisch in x- und y-Richtung einer horizontalen Positionierungsebene relativ zur Basisstation 2 bewegt werden kann. Die translatorische Bewegung erfolgt mittels translatorischer Bewegungstreiber oder manuell über entsprechende Führungen.

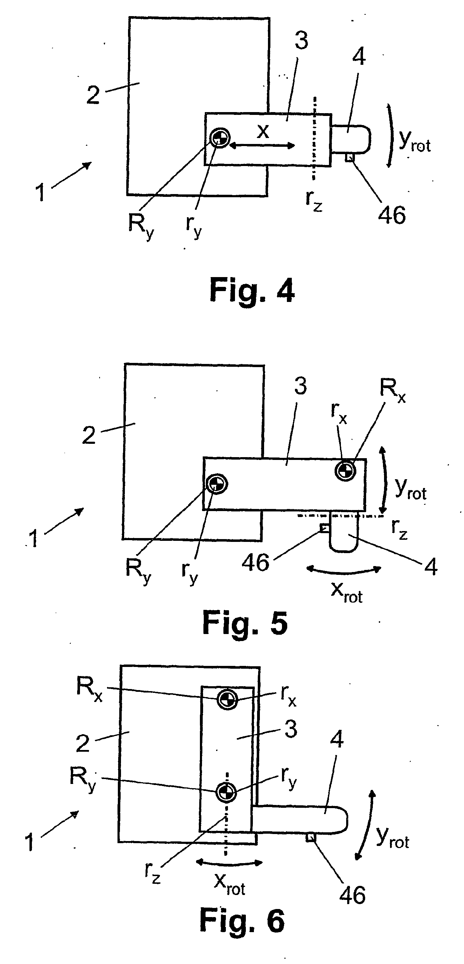

In der Ausführungsvariante nach Figur 4 ist der Tragarm 3 über das Drehgelenk R_{y} mit der Basisstation 2 verbunden. Das Drehgelenk R_{y} ermöglicht eine Drehung yᵣₒₜ des Tragarms 3 um die vertikale Drehachse r_{y}. Wie in der Figur 4 schematisch dargestellt ist, ist das Drehgelenk R_{y} so angeordnet und ausgeführt, dass durch die Drehung yᵣₒₜ des Tragarms 3 um die Drehachse r_{y} eine horizontale Bewegung in der y-Richtung zum Ausrichten des Lichtprojektors 41 zum Auge 6 ermöglicht wird. Die Ausrichtung des Lichtprojektors 41 in der x-Richtung erfolgt wie in den Figuren 2 und 3 mittels translatorischer Bewegung.

In der Ausführungsvariante nach Figur 5 erfolgt die Ausrichtung des Lichtprojektors 41 sowohl in der x- als auch in der y-Richtung rotatorisch. In der Ausführungsvariante nach Figur 5 ist der Applikationskopf 4 insbesondere auch über das Drehgelenk Rₓ mit dem von der Basisstation 2 abgewandten Ende des Tragarms 3 verbunden. Das Drehgelenk Rₓ ermöglicht eine Drehung xᵣₒₜ des Applikationskopfs 4 um die vertikale Drehachse rₓ. Wie in der Figur 5 schematisch dargestellt ist, ist das Drehgelenk Rₓ so angeordnet und ausgeführt, dass durch die Drehung xᵣₒₜ des Applikationskopfs 4 um die Drehachse rₓ eine horizontale Bewegung in der x-Richtung zum Ausrichten des Lichtprojektors 41 zum Auge 6 ermöglicht wird. Die Ausrichtung des Lichtprojektors 41 in der y-Richtung erfolgt wie in der Figur 4 mittels rotatorischer Bewegung.

In der Ausführungsvariante nach Figur 6 erfolgt die Ausrichtung des Lichtprojektors 41, wie in der Figur 5, sowohl in der x- als auch in der y-Richtung rotatorisch. In der Ausführungsvariante nach Figur 6 ist jedoch das Drehgelenk R_{z} respektive die Drehachse r_{z} bezüglich der Anordnung gemäss Figur 5 um 90° gedreht. Die Bewegung in der x-Richtung erfolgt durch eine Drehung xᵣₒₜ des Tragarms 3 um die vertikale Drehachse rₓ des Drehgelenks Rₓ, das den Tragarm 3 mit der Basisstation 2 verbindet. Die Bewegung in der y-Richtung erfolgt durch eine Drehung yᵣₒₜ des Applikationskopfs 4 um die vertikale Drehachse r_{y} des Drehgelenks R_{y}, das den Applikationskopf 4 mit dem Tragarm 3 verbindet.

Die Positionierungsmittel zum rotatorischen und/oder translatorischen Bewegen des Tragarms 3 für die horizontale Ausrichtung des Applikationskopfs 4 respektive des Lichtprojektors 41 mit translatorischen Verschiebungen in der x- und y-Richtung und/oder mit rotatorischen Drehungen xᵣₒₜ und yᵣₒₜ können für die manuelle Bewegung und/oder mittels Bewegungstreibern ausgeführt werden.

Obwohl dies nur in der Figur 2 schematisch dargestellt ist, umfasst die ophthalmologische Vorrichtung 1 Mittel zur Gewichtskompensation 33, die mit dem Applikationskopf 4 verbunden sind, beispielsweise einstellbare Gegengewichte oder Federn. Die Mittel zur Gewichtskompensation 33 sind vorzugsweise so ausgestaltet, dass sie die mit dem Applikationskopf 4 um die Drehachse r_{z} drehbaren Massen nur teilweise austarieren, so dass der Applikationskopf 4 mit einer definierten Applikationskraft auf das Auge 6 aufsetzbar ist.

Obwohl in den Figuren und den oben stehenden Ausführungen bloss Ausführungsvarianten angeführt wurden, in denen der Applikationskopf 4 zur Ausführung der vertikalen Ausrichtung in z-Richtung über das Drehgelenk R_{z} mit dem Tragarm 3 verbunden ist, soll hier festgehalten werden, dass die vertikale Ausrichtung des Applikationskopfs 4 in z-Richtung auch über eine Drehbewegung des Tragarms 3 um eine horizontale Drehachse r_{z} ausgeführt werden kann, wenn der Tragarm 3 über ein entsprechendes Drehgelenk R_{z} mit der Basisstation 2 verbunden ist, und der Applikationskopf 4 fest am Tragarm 3 angebracht ist. Die horizontale Ausrichtung des Applikationskopfs 4 respektive des Lichtprojektors 41 mit translatorischen Verschiebungen in der x- und y-Richtung und/oder mit rotatorischen Drehungen xᵣₒₜ und yᵣₒₜ erfolgt ebenfalls durch die Positionierungsmittel zum rotatorischen und/oder translatorischen Bewegen des Tragarms 3 entsprechend der oben stehenden Beschreibung. Je nach Anordnung der Positionierungsmittel zum rotatorischen und/oder translatorischen Bewegen des Tragarms 3 bleibt das Drehgelenk R_{z} dabei fest mit der Basisstation 2 verbunden oder wird mitbewegt.

Obwohl dies nur in der Figur 1 schematisch dargestellt ist, umfasst die ophthalmologische Vorrichtung 1 zudem eine in der Basisstation 2 angeordnete Steuereinheit 23. Die Steuereinheit 23 stellt sicher, dass die Strahlablenkungsmittel 31 nicht aktiviert sind, wenn der Applikationskopf 4 zum Aufsetzen auf das Auge 6 bewegt wird. Die Steuereinheit 23 ist überdies eingerichtet für die Ansteuerung der Strahlablenkung sowie die Steuerung und Überwachung von Bewebungstreibem. Die Steuereinheit 23 umfasst zudem Sicherheitsfunktionen zur Überwachung von Kräften, Bewegungen und Strahlparametern.

In einer Ausführungsvariante umfasst die ophthalmologische Vorrichtung 1 überdies Höhenbestimmungsmittel 22 zum Ermitteln einer vertikalen Lage des Auges 6, beispielsweise eine Kamera, und die Basisstation 2 umfasst Höhenpositionierungsmittel 24, z.B. translatorische Bewegungstreiber, zum Einstellen einer vertikalen Grundposition der Basisstation 2 respektive des damit verbundenen Tragarms 3. Die Höhenpositionierungsmittel 24 werden beispielsweise durch die Steuereinheit 23 auf Grund einer durch die Höhenbestimmungsmittel 22 ermittelten vertikalen Lage des Auges 6 gesteuert. Es ist auch eine manuelle Einstellung der Grundposition möglich. Durch Einstellung der Grundposition kann' der für die Applikation benötigte Bewegungshub für den Applikationskopf 4 auf beispielsweise 10-20mm reduziert werden.

## Patentansprüche

1. Ophthalmologische Vorrichtung (1), umfassend:
eine Basisstation (2) mit einer Lichtquelle (21) zur Erzeugung von Lichtpulsen,
einen an der Basisstation (2) angebrachten Tragarm (3),
einen am Tragarm (3) angebrachten und auf ein Auge (6) aufsetzbaren Applikationskopf (4) mit einem Lichtprojektor (41) zur fokussierten Projektion der Lichtpulse für eine punktuelle Auflösung von Augengewebe, und
ein optisches Übertragungssystem (22) zur Übertragung der Lichtpulse von der Basisstation (2) durch den Tragarm (3) zum Applikationskopf (4),
wobei der Tragarm (3) starr ausgeführt ist und den Applikationskopf (4) mit der Basisstation (2) verbindet, und
der Tragarm (3) an einem Ende ein Drehgelenk (R_{z}) mit einer horizontal ausgerichteten Drehachse (r_{z}) aufweist,
wobei das Drehgelenk (R_{z}) so angebracht ist, dass der Applikationskopf (4) mit einer um die horizontale Drehachse (r_{z}) verlaufenden Drehung (zᵣₒₜ) manuell auf das Auge (6) aufsetzbar ist,
**dadurch gekennzeichnet,**
**dass** im Tragarm (3) Strahlablenkungsmittel (31) angeordnet sind, welche eingerichtet sind, die Lichtpulse in mindestens zwei Abtastrichtungen abzulenken, wobei die abgelenkten Lichtpulse durch den Lichtprojektor (41) zur Auflösung von Augengewebe fokussiert projizierbar sind.

2. Vorrichtung (1) nach Anspruch 1, **dadurch gekennzeichnet, dass** das Drehgelenk (R_{z}) am von der Basisstation (2) abgewandten Ende des Tragarms (3) angeordnet ist, und dass der Applikationskopf (4) über das Drehgelenk (R_{z}) beweglich mit dem Tragarm (3) verbunden ist.

3. Vorrichtung (1) nach Anspruch 1, **dadurch gekennzeichnet, dass** der Applikationskopf (4) fest mit dem Tragarm (3) verbunden ist, dass das Drehgelenk (R_{z}) am der Basisstation (2) zugewandten Ende des Tragarms (3) angeordnet ist, und dass der Tragarm (3) über das Drehgelenk (R_{z}) beweglich mit der Basisstation (2) verbunden ist.

4. Vorrichtung (1) nach einem der Ansprüche 1 bis 3, **dadurch gekennzeichnet, dass** die Vorrichtung (1) Positionierungsmittel umfasst zum rotatorischen und/oder translatorischen Bewegen des Tragarms (3) parallel zu einer Positionierungsebene für die Positionierung des Applikationskopfs (4) über das Auge (6).

5. Vorrichtung (1) nach einem der Ansprüche 1 bis 4, **dadurch gekennzeichnet, dass** der Lichtprojektor (41) so dimensioniert ist, dass abgelenkte Lichtpulse ohne mechanische Bewegung des Lichtprojektors (41) über einen gesamten Arbeitsbereich zur Auflösung von Augengewebe fokussiert projizierbar sind.

6. Vorrichtung (1) nach einem der Ansprüche 1 bis 5, **dadurch gekennzeichnet, dass** der Applikationskopf (4) ein Sichtfenster (44) aufweist, das eine Aufsicht auf das Auge (6) in Projektionsrichtung (v) des Lichtprojektors (41) ermöglicht.

7. Vorrichtung (1) nach Anspruch 6, **dadurch gekennzeichnet, dass** die Vorrichtung (1) eines oder mehrere beweglich mit der Basisstation (2) verbundene optische Sehhilfe- oder Messmodule (5) umfasst, die In Projektionsrichtung (v) des Lichtprojektors (41) ausgerichtet über das Sichtfenster (44) schwenkbar sind.

8. Vorrichtung (1) nach einem der Ansprüche 1 bis 7, **dadurch gekennzeichnet, dass** Mittel zur Gewichtskompensation (33) mit dem Applikationskopf (4) verbunden sind, um den Applikationskopf (4) über der Drehachse (r_{z}) teilweise so auszubalancieren, dass der Applikationskopf (4) mit einer vom Eigengewicht des Applikationskopfs (4) reduzierten Applikationskraft auf das Auge (6) aufsetzbar ist.

9. Vorrichtung (1) nach einem der Ansprüche 1 bis 8, **dadurch gekennzeichnet, dass** der Lichtprojektor (41) ein erstes Linsensystem (43) in einem in Projektionsrichtung (v) ausgerichteten Projektionsteil (4B) des Applikationskopfs (4) umfasst, und dass der Lichtprojektor (41) ein zweites Linsensystem (42) in einem vom Projektionsteil (4B) abgewinkelten, dem Tragarm (3) zugewandten Zuführteil (4A) des Applikationskopfs (4) umfasst, wobei das erste und das zweite Linsensystem über einen Umlenkspiegel (45) gekoppelt sind.

10. Vorrichtung (1) nach einem der Ansprüche 1 bis 9, **dadurch gekennzeichnet, dass** der Applikationskopf (4) einen in Projektionsrichtung (v) ausgerichteten Projektionsteil (4B) umfasst, dass der Applikationskopf (4) einen vom Projektionsteil (4B) abgewinkelten, dem Tragarm (3) zugewandten Zuführteil (4A) umfasst, und dass der Projektionsteil (4B) drehbar mit dem Zuführteil (4A) verbunden ist.

11. Vorrichtung (1) nach einem der Ansprüche 1 bis 10, **dadurch gekennzeichnet, dass** die Vorrichtung (1) eine Verriegelung (32) umfasst zum Fixieren respektive Lösen einer Drehstellung des Applikationskopf (4) um die Drehachse (r_{z}).

12. Vorrichtung (1) nach einem der Ansprüche 1 bis 11, **dadurch gekennzeichnet, dass** die Vorrichtung (1) Höhenbestimmungsmittel (22) zum Ermitteln einer vertikalen Lage des Auges (6) umfasst, und dass die Basisstation (2) Höhenpositionierungsmittel (24) umfasst zum Einstellen einer vertikalen Grundposition des Tragarms (3).

13. Vorrichtung (1) nach einem der Ansprüche 1 bis 12, **dadurch gekennzeichnet, dass** der Applikationskopf (4) mindestens eines aus Griffen und Griffstrukturen (46) aufweist, dass der Applikationskopf (4) einen auf das Auge (6) aufsetzbaren, mindestens stellenweise lichtdurchlässigen Kontaktkörper umfasst, welcher so ausgebildet und angeordnet ist, dass er einen im aufgesetzten Zustand kontaktierten Bereich des Auges (6) äquidistant zu einer Arbeitsfläche setzt, dass der Applikationskopf (4) Befestigungsmittel aufweist zum Fixieren des Applikationskopfs (4) durch Unterdruck am Auge (6), und dass die Lichtquelle (21) einen Femtosekundenlaser umfasst.

## Claims

1. Ophthalmological apparatus (1), comprising:
a base station (2) with a light source (21) for generating light pulses,
a support arm (3) mounted on the base station (2),
an application head (4) that is mounted on the support arm (3) and can be placed onto an eye (6) and has a light projector (41) for focused projection of the light pulses for punctiform breakdown of eye tissue, and
an optical transmission system (22) for transmitting the light pulses from the base station (2) through the support arm (3) to the application head (4),
the support arm (3) being of rigid design and connecting the application head (4) to the base station (2), and
the support arm (3) having, at one end, a hinge (R_{z}) with a horizontally oriented rotation axis (r_{z}),
the hinge (R_{z}) being mounted in such a way that the application head (4) can be placed manually onto the eye (6) with a rotation (zᵣₒₜ) extending about the horizontal rotation axis (r_{z}),
**characterized in that**
beam-deflecting means (31) are arranged in the support arm (3) and are designed to deflect the light pulses in at least two scanning directions, the deflected light pulses being able to be projected in a focused manner through the light projector (41) for breakdown of eye tissue.

2. Apparatus (1) according to Claim 1, **characterized in that** the hinge (R_{z}) is arranged on that end of the support arm (3) directed away from the base station (2), and **in that** the application head (4) is connected movably to the support arm (3) via the hinge (R_{z}).

3. Apparatus according to Claim 1, **characterized in that** the application head (4) is connected fixedly to the support arm (3), **in that** the hinge (R_{z}) is arranged on that end of the support arm (3) directed towards the base station (2), and **in that** the support arm (3) is connected movably to the base station (2) via the hinge (R_{z}).

4. Apparatus (1) according to one of Claims 1 to 3, **characterized in that** the apparatus (1) comprises positioning means for rotary and/or translatory movement of the support arm (3) parallel to a positioning plane for the positioning of the application head (4) over the eye (6).

5. Apparatus (1) according to one of Claims 1 to 4, **characterized in that** the light projector (41) is dimensioned such that, without mechanical movement of the light projector (41), deflected light pulses can be projected in a focused manner across an entire work area for breakdown of eye tissue.

6. Apparatus (1) according to one of Claims 1 to 5, **characterized in that** the application head (4) has a viewing window (44) permitting a top view onto the eye (6) in the projection direction (v) of the light projector (41).

7. Apparatus (1) according to Claim 6, **characterized in that** the apparatus (1) comprises one or more optical viewing or measuring modules (5) which are connected movably to the base station (2) and which, oriented in the projection direction (v) of the light projector (41), can be swivelled over the viewing window (44).

8. Apparatus (1) according to one of Claims 1 to 7, **characterized in that** weight compensation means (33) are connected to the application head (4), in order to partially balance out the application head (4) above the rotation axis (r_{z}) in such a way that the application head (4) can be placed onto the eye (6) with an application force reduced by the inherent weight of the application head (4).

9. Apparatus (1) according to one of Claims 1 to 8, **characterized in that** the light projector (41) comprises a first lens system (43) in a projection part (4B) of the application head (4) oriented in the projection direction (v), and **in that** the light projector (41) comprises a second lens system (42) in a feed part (4A) of the application head (4) angled away from the projection part (4b) and directed towards the support arm (3), said first and second lens systems being coupled via a deflecting mirror (45).

10. Apparatus (1) according to one of Claims 1 to 9, **characterized in that** the application head (4) comprises a projection part (4B) oriented in the projection direction (v), **in that** the application head (4) comprises a feed part (4A) angled away from the projection part (4B) and directed towards the support arm (3), and **in that** the projection part (4B) is connected rotatably to the feed part (4A).

11. Apparatus (1) according to one of Claims 1 to 10, **characterized in that** the apparatus (1) comprises a lock (32) for fixing or releasing a rotation position of the application head (4) about the rotation axis (r_{z}).

12. Apparatus (1) according to one of Claims 1 to 11, **characterized in that** the apparatus (1) comprises height-defining means (22) for determining a vertical position of the eye (6), and **in that** the base station (2) comprises height-positioning means (24) for setting a vertical basic position of the support arm (3).

13. Apparatus (1) according to one of Claims 1 to 12, **characterized in that** the application head (4) has at least one of grips and grip structures (46), **in that** the application head (4) comprises a contact body which can be placed onto the eye (6), is transparent to light at least in parts and is configured and arranged such that it sets a contacted area of the eye (6) equidistant to a work surface, **in that** the application head (4) has securing means for fixing the application head (4) on the eye (6) by underpressure, and **in that** the light source (21) comprises a femtosecond laser.

## Revendications

1. Dispositif ophtalmologique (1) comprenant :
un poste de base (2) doté d'une source de lumière (21) permettant de former des impulsions lumineuses,
un bras de support (3) installé sur le poste de base (2),
une tête d'application (4) installée sur le bras de support (3), apte à être placée sur un oeil (6) et dotée d'un projecteur de lumière (41) qui permet de projeter de manière focalisée l'impulsion lumineuse en vue de détacher ponctuellement le tissu oculaire et
un système optique de transmission (22) qui transmet les impulsions lumineuses du poste de base (2) à la tête d'application (4) par l'intermédiaire du bras de support (3),
le bras de support (3) étant rigide et reliant la tête d'application (4) au poste de base (2),
le bras de support (3) présentant à une extrémité une articulation rotative (R_{z}) dont l'axe de rotation (r_{z}) est orienté à l'horizontale,
l'articulation rotative (R_{z}) étant installée de telle sorte que la tête d'application (4) puisse être placée manuellement sur l'oeil (6) par une rotation (Zᵣₒₜ) autour de l'axe horizontal de rotation (r_{z}),
**caractérisé en ce que**
des moyens (31) de déviation de faisceau sont disposés sur le bras de support (3) et sont conçus de manière à dévier l'impulsion lumineuse dans au moins deux directions de palpage, les impulsions lumineuses déviées pouvant être projetées en étant focalisées par le projecteur de lumière (41) pour détacher le tissu oculaire.

2. Dispositif (1) selon la revendication 1, **caractérisé en ce que** l'articulation rotative (R_{z}) est disposée à l'extrémité du bras de support (3) non tournée vers le poste de base (2) et **en ce que** la tête d'application (4) est reliée à déplacement au bras du support (3) par l'intermédiaire de l'articulation rotative (R_{z}).

3. Dispositif (1) selon la revendication 1, **caractérisé en ce que** la tête d'application (4) est reliée solidairement au bras de support (3), **en ce que** l'articulation rotative (R_{z}) est disposée à l'extrémité du bras de support (3) tournée vers le poste de base (2) et **en ce que** le bras de support (3) est relié à déplacement au poste de base (2) par l'intermédiaire de l'articulation rotative (R_{z}).

4. Dispositif (1) selon l'une des revendications 1 à 3, **caractérisé en ce que** le dispositif (1) comporte des moyens de positionnement qui permettent de déplacer le bras de support (3) en rotation et/ou en translation parallèle à un plan de positionnement utilisé pour positionner la tête d'application (4) au-dessus de l'oeil (6).

5. Dispositif (1) selon l'une des revendications 1 à 4, **caractérisé en ce que** le projecteur de lumière (41) est dimensionné de telle sorte que les impulsions lumineuses déviées puissent être projetées de manière focalisée sans déplacement mécanique du projecteur de lumière (41) sur la totalité de la plage de travail utilisée pour détacher le tissu oculaire.

6. Dispositif (1) selon l'une des revendications 1 à 5, **caractérisé en ce que** la tête d'application (4) présente une fenêtre d'observation (44) qui permet de voir l'oeil (6) dans la direction de projection (v) du projecteur de lumière (41).

7. Dispositif (1) selon la revendication 6, **caractérisé en ce que** le dispositif (1) présente plusieurs modules auxiliaires d'observation ou de mesure optique (5) reliés à déplacement au poste de base (2) et aptes à pivoter au-dessus de la fenêtre d'observation (44) en alignement sur la direction de projection (v) du projecteur de lumière (41).

8. Dispositif (1) selon l'une des revendications 1 à 7, **caractérisé en ce que** des moyens (33) de compensation du poids sont reliés à la tête d'application (4) pour équilibrer partiellement la tête d'application (4) par rapport à l'axe de rotation (r_{z}) de telle sorte que la tête d'application (4) puisse être placée sur l'oeil (6) avec une force d'application moindre que celle du poids propre de la tête d'application (4).

9. Dispositif (1) selon l'une des revendications 1 à 8, **caractérisé en ce que** le projecteur de lumière (41) présente un premier système (43) de lentilles dans une partie de projection (48) de la tête d'application (4) orientée dans la direction de projection (v) et **en ce que** le projecteur de lumière (41) présente un deuxième système (42) de lentilles dans une partie (4A) d'amenée de la tête d'application (4) tournée vers le bras de support (3) et oblique par rapport à la partie de projection (4B), le premier et le deuxième système de lentilles pouvant être couplés par l'intermédiaire d'un miroir de renvoi (45).

10. Dispositif (1) selon l'une des revendications 1 à 9, **caractérisé en ce que** la tête d'application (4) présente une partie de projection (4B) orientée dans la direction de projection (v), **en ce que** la tête d'application (4) présente une deuxième partie d'amenée (4A) tournée vers le bras de support (3) et oblique par rapport à la partie de projection (4B) et **en ce que** la partie de projection (48) est reliée à rotation à la partie d'amenée (4A).

11. Dispositif (1) selon l'une des revendications 1 à 10, **caractérisé en ce que** le dispositif (1) présente un verrouillage (32) qui immobilise ou libère la position de rotation de la tête d'application (4) autour de l'axe de rotation (r_{z}).

12. Dispositif (1) selon l'une des revendications 1 à 11, **caractérisé en ce que** le dispositif (1) présente des moyens (22) de détermination de hauteur qui déterminent la position verticale de l'oeil (6) et **en ce que** le poste de base (2) présente des moyens (24) de positionnement en hauteur qui ajustent la position fondamentale du bras de support (3) dans la direction verticale.

13. Dispositif (1) selon l'une des revendications 1 à 12, **caractérisé en ce que** la tête d'application (4) présente au moins une poignée ou structure de poignée (46), **en ce que** la tête d'application (4) présente un corps de contact apte à être placé sur l'oeil (6), dont au moins certaines parties sont transparentes, configuré et disposé de manière à placer en position équidistante par rapport à une surface de travail une partie de l'oeil (6) qui est en contact avec lui lorsqu'il est placé, **en ce que** la tête d'application (4) présente des moyens de fixation qui immobilisent la tête d'application (4) par dépression sur l'oeil (6) et **en ce que** la source de lumière (21) comporte un laser travaillant à une fréquence de l'ordre des femtosecondes.
